# DEMANDE DE BREVET EUROPEEN

(11) **EP 1 118 347 A1**
(43) Date de publication de la demande: **25.07.2001**
(21) Numéro de dépôt: 01400027.7
(22) Date de dépôt: 08.01.2001
(51) Int. Cl.: A61M 16/20

(54) **Appareil d'anesthésie respiratoire à valve de décharge pilotée**

(30) Priorité: 21.01.2000 FR 0000781
(71) Demandeur: TAEMA, F-92182 Antony Cédex (FR)
(72) Inventeur: Kitten, Sébastien, 91160 Saulx les Charteux (FR)
(74) Mandataire: Pittis, Olivier

(57) **Abrégé**

Appareil d'anesthésie respiratoire comportant un circuit de gaz (1) comprenant une branche inspiratoire pour acheminer un mélange de gaz anesthésique jusqu'à des moyens de liaison (24) destinés à être reliés aux voies aériennes supérieures d'un patient (11) ; une branche expiratoire (3) pour acheminer un mélange gazeux contenant du CO₂ expiré par ledit patient (11); lesdites branche inspiratoire (2) et expiratoire (3) formant un circuit de gaz (1) en boucle. Une ligne (8) d'alimentation en gaz frais, en communication fluide avec le circuit (1) de gaz est destinée à alimenter ledit circuit (1) en gaz frais. En outre, une valve (4) de décharge à valeur de consigne de pression ajustable est agencée sur la branche inspiratoire (2) ou expiratoire (3) et des moyens de pilotage de la valve de décharge (4) agissent sur la valve de décharge (4) en réponse à la détection d'une variation de débit et/ou de pression du gaz frais à l'intérieur de la ligne (8) d'alimentation en gaz frais pour ajuster la consigne de pression de la valve (4) de décharge.

## Description

La présente invention concerne un appareil d'anesthésie respiratoire comportant un dispositif permettant de réduire la constante de temps du circuit patient par asservissement de la soupape de décharge dudit circuit patient.

De manière connue en soi, les appareils d'anesthésie fonctionnent classiquement en circuit fermé.

En effet, ces appareils comportent généralement des dispositifs de récupération du gaz expiré par le patient, de manière à assurer une élimination du CO₂ qui s'y trouve et son renvoi vers le patient durant une phase inspiratoire subséquente.

Par ailleurs, les appareils d'anesthésie comportent un dispositif permettant d'assurer une ventilation du patient en lui administrant un gaz qui contient des vapeurs anesthésiques.

Le patient consomme une partie du gaz inhalé et expire le reste.

Les gaz expirés par le patient sont récupérés, débarrassés du CO₂ pour être renvoyés vers le patient. La consommation du patient est compensée par un apport en gaz frais. De plus, les défauts d'étanchéité du circuit créent un déficit en gaz pendant l'insufflation et l'expiration. Ce déficit est également compensé par l'apport de gaz frais.

Les concentrations gazeuses du mélange insufflé au patient varient selon la prescription médicale, au cours de l'anesthésie. Lors de ces changements de concentration, le praticien augmente l'apport en gaz frais, alors qu'un trop plein est évacué par une valve spécifique.

En jouant sur le débit de gaz frais, on peut directement augmenter ou diminuer la vitesse de modification des concentrations dans le circuit. Il en résulte que la consommation en gaz frais peut être fortement augmentée, ce qui induit un surcoût important et entraîne une pollution accrue du bloc opératoire par les vapeurs anesthésiques.

Des ventilateurs d'anesthésie de ce type sont décrits dans les documents EP-A-761249 et EP-A-745404.

En d'autres termes, compte tenu de la diversité des situations rencontrées au cours d'une intervention chirurgicale, médicale ou analogue mettant en oeuvre une anesthésie respiratoire du patient, obtenue à l'aide d'un appareil d'anesthésie respiratoire, l'opérateur de l'appareil, par exemple un médecin, un infirmier ou analogue, est parfois contraint de modifier les modalités de la ventilation du patient notamment de faire varier la quantité de gaz frais envoyé dans le circuit patient, en particulier, dans la branche inspiratoire de ce circuit patient.

Pour ce faire, les appareils d'anesthésie fonctionnant en circuit fermé avec réinhalation des gaz sont alimentés par un apport d'un débit sensiblement continu de gaz frais et mettent en oeuvre une valve de décharge pour limiter la pression interne du circuit patient.

Habituellement, la valve de décharge ou soupape de décharge est agencée dans la branche inspiratoire ou dans la branche expiratoire du circuit patient.

Cette valve de décharge permet d'éviter toute élévation intempestive de la pression du gaz dans le circuit patient, au-delà d'une valeur de pression de consigne préfixée.

Cependant, dans le cas de débits de gaz importants, notamment pendant les phases de changement de concentration, il a été constaté, en pratique, que la valve de décharge maintenait quasiment toujours une pression nettement supérieure à la pression nominale de consigne, ceci étant dû aux pertes de charges de l'ensemble du système.

En d'autres termes, aucun système actuel ne prend en compte les influences du débit et/ou de la pression de gaz frais sur la pression résiduelle du circuit induit par la valve de décharge et par voie de conséquence la pression résiduelle au niveau des poumons du patient.

Actuellement, lorsque l'utilisateur souhaite modifier la composition du mélange gazeux présent dans le circuit grâce à une forte augmentation du débit de gaz frais à une nouvelle concentration, il modifie la valeur d'ouverture de la valve de décharge, faisant ainsi monter en pression le circuit patient.

Or, cette augmentation de pression engendre alors une augmentation de la pression dans les poumons du patient, ce qui a pour effet de réduire le passage du sang par les poumons et réduire , par là-même, l'efficacité des variations de concentration des gaz inhalés, ce qui est contraire à l'objectif recherché.

En outre, la pression dans le circuit induite par la pression d'ouverture de la valve de décharge sert à maintenir gonflé l'organe d'accumulation qui sert à la récupération des gaz expirés. Cet organe d'accumulation participe à la dilution des gaz gas frais dans le circuit patient et son volume interne introduit directement un retard dans la modification des concentrations.

Ainsi, on comprend aisément qu'une diminution de la pression d'ouverture de la valve de décharge permet notamment de diminuer le volume de l'organe d'accumulation et d'améliorer la constante de temps du circuit sur les variations de concentration, alors qu'en fait, l'augmentation du débit de gaz frais produit l'effet contraire sur la valve de décharge.

Il en résulte alors que l'augmentation du débit de gaz frais engendre des effets indirects contraires à l'objectif recherché.

Ainsi, la seule solution dont l'opérateur dispose pour accélérer le temps de réponse du système aux variations de concentration est d'augmenter considérablement le débit de gaz frais puisqu'une augmentation légère du débit en suffit pas.

L'utilisateur provoque ainsi un rejet à l'atmosphère de gaz frais et donc de produits halogénés servant à l'anesthésie.

Le but de la présente invention est alors de résoudre les problèmes susmentionnés, en proposant un appareil d'anesthésie respiratoire permettant de prendre en compte la valeur du débit de gaz frais et de l'intégrer dans la commande de la valve de décharge, de manière à gagner sur la constante de temps du circuit et donc d'économiser des gaz frais, en particulier des composés halogénés, sans créer d'effets secondaires sur le patient qui iraient à l'encontre de la stratégie de soin mise en place par le médecin.

La présente invention concerne alors un appareil d'anesthésie respiratoire comportant :
- circuit de gaz comprenant :
   (a) une branche inspiratoire susceptible d'acheminer un mélange de gaz anesthésique jusqu'à des moyens de liaison destinés à être reliés aux voies aériennes supérieures d'un patient ;
   (b) une branche expiratoire susceptible d'acheminer un mélange gazeux contenant du CO₂ expiré par ledit patient ;
      lesdites branche inspiratoire et branche expiratoire formant le circuit de gaz en boucle,
- au moins une ligne d'alimentation en gaz frais, en communication fluide avec au moins une partie dudit circuit de gaz, et destinée à alimenter ledit circuit de gaz en gaz frais ;
- au moins une valve de décharge à valeur de consigne de pression ajustable, agencée sur la branche inspiratoire ou sur la branche expiratoire ; et
- des moyens de pilotage de la valve de décharge agissant sur ladite valve de décharge en réponse à la détection d'une variation de débit et/ou de pression du gaz frais à l'intérieur d'au moins une partie de la ligne d'alimentation en gaz frais pour ajuster la consigne de pression de ladite valve de décharge.

Selon le cas, l'appareil peut comprendre l'une ou plusieurs des caractéristiques suivantes :
- les moyens de pilotage agissent en réponse à une variation d'au moins le débit dans au moins une partie de la ligne d'alimentation.
- les moyens de pilotage comprennent des moyens de mesure de débit et/ou de pression agencés sur la ligne d'alimentation en gaz frais et des moyens de traitement électronique reliés électriquement auxdits moyens de mesure.
- les moyens de traitement électronique agissent sur la valve de décharge en réponse auxdits moyens de mesure.
- les moyens de traitement commandent une diminution de la valeur de consigne de pression de la valve de décharge en réponse à une détermination par les moyens de mesure, d'une augmentation de débit de gaz frais dans la ligne d'alimentation.
- les moyens de traitement agissent sur la valve de décharge par l'intermédiaire d'une ligne de commande de pression.
- les moyens de pilotage comprennent un dispositif à venturi agencé dans la ligne d'alimentation en gaz frais, ledit dispositif à venturi étant relié pneumatiquement à un dispositif d'équilibrage de pression, ledit dispositif d'équilibrage étant relié pneumatiquement à ladite valve de décharge.
- ledit dispositif d'équilibrage de pression comporte au moins deux chambres internes séparées par une cloison mobile et/ou déformable.

En outre, l'invention concerne aussi un procédé de commande d'un appareil selon l'invention, caractérisé en ce qu'on procède selon les étapes de :
(a) déterminer au moins une information de débit et/ou pression de gaz frais circulant dans la ligne d'alimentation au moyen des moyens de mesure de débit et/ou de pression agencés sur ladite ligne ;
(b) transmettre ladite information auxdits moyens de traitement électroniques et traiter ladite information ;
(c) modifier ou ajuster la valeur de pression de consigne de la valve de décharge.

De préférence, le traitement de l'information de l'étape (b) comprend une intégration de la valeur de débit mesurée à l'étape (a) et une comparaison de cette valeur intégrée à une valeur de référence.

Avantageusement, on diminue la valeur de la pression de consigne de la valve de décharge lorsqu'on détermine que le débit de gaz frais mesuré augmente.

L'invention va maintenant être décrite plus en détail à l'aide des figures annexées représentant deux modes de réalisation d'un appareil selon l'invention, donnés à titre illustratif mais non limitatif.

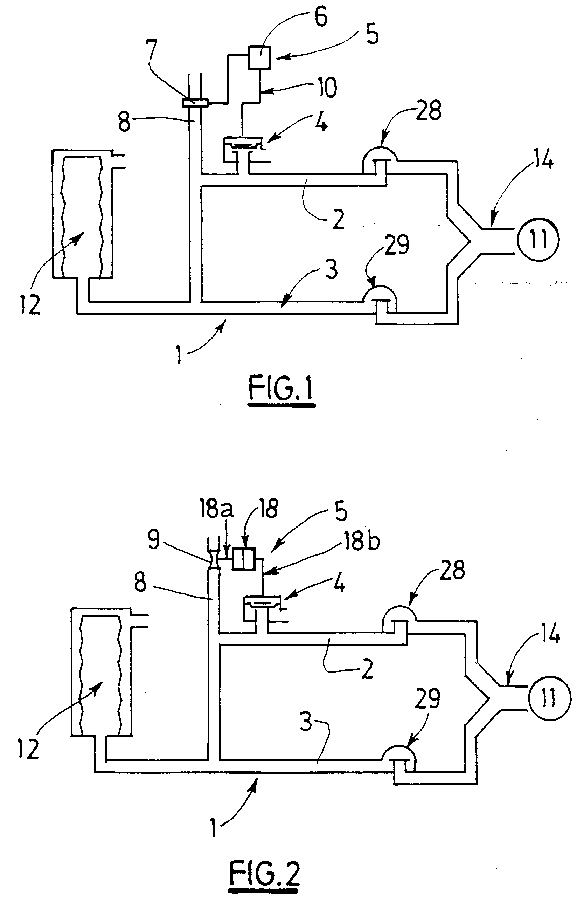

La figure 1 représente un schéma d'un appareil d'anesthésie respiratoire selon l'invention, lequel appareil comporte un circuit de gaz en boucle 1 formé, d'une part, d'une branche inspiratoire 2 et d'une branche expiratoire 3.

La branche inspiratoire 2 achemine un gaz respiratoire anesthésique depuis une source 12 de gaz respiratoire anesthésique 10, ici un réservoir d'accumulation utilisé lors d'une ventilation en mode machine, jusqu'à des moyens de liaisons 14 reliés aux voies aériennes supérieures d'un patient 11, lesdits moyens de liaisons 14 pouvant être un masque respiratoire, des sondes d'intubation ou tout moyen analogue.

A l'inverse, la branche expiratoire 3 permet de recueillir les gaz expirés par le patient 11 durant les phases expiratoires, lesdits gaz expirés étant riches en dioxyde de carbone (CO₂) et contenant des produits anesthésiques tels des halogénés, qu'il est souhaitable de pouvoir récupérer et recycler en vue de leur renvoi ultérieur vers le patient 11.

Lesdites branches inspiratoires 2 et expiratoires 3 forment un circuit en boucle 1, encore appelé circuit fermé ou circuit patient.

La branche inspiratoire 2 est équipée d'une valve 4 d'échappement à l'atmosphère permettant de palier toute surpression au sein de ladite branche inspiratoire, laquelle surpression pourrait être néfaste pour le patient 11. Cette valve 4 d'échappement est communément appelée valve de décharge ou soupape de décharge.

Par ailleurs, la branche expiratoire 3 comporte classiquement des moyens de purification (non schématisés), tel un dispositif d'épuration contenant un absorbant, par exemple, de la chaux ou analogue, destiné à éliminer la majeure partie du CO₂ contenu dans les gaz expirés par le patient.

En outre, la branche inspiratoire 2 et la branche expiratoire 3 sont équipées de clapets ou valves anti-retour 28 et 29, respectivement.

Le mode de réalisation de la figure 1 comprend, en outre, des moyens de pilotage de type électronique agissant en réponse à une augmentation du débit de gaz frais dans la ligne 8 pour modifier, si nécessaire, en conséquence la valeur de pression de consigne de la valve de décharge 4.

Plus précisément, ces moyens de pilotage comprennent des moyens de mesure de débit 7, tel un capteur de débit, agencés sur la ligne 8 et reliés électriquement à des moyens de traitement 6 électronique, telle une carte à microprocesseur de manière à pouvoir transmettre auxdits moyens de traitement 6, la valeur de débit mesurée dans la ligne 8.

Les moyens de traitement 6 traitent alors la valeur de débit et permettent de déterminer, selon une table préétablie, la valeur de pression de consigne de la valve de décharge 4.

En d'autres termes, le dispositif de l'invention permet de mesurer le débit de gaz frais et d'intégrer la valeur de débit mesurée pour modifier la consigne de pression appliquée sur la valve 4 de décharge du circuit 1.

En outre, la figure 2 représente un second mode de réalisation d'un appareil d'anesthésie selon l'invention, laquelle est analogue à la figure 1, à l'exception du fait que, dans ce cas, la commande de la valve 4 de décharge est effectuée par le biais d'un dispositif du type pneumatique.

Plus précisément, ce dispositif de type pneumatique comprend un dispositif à venturi 9 agencé sur le passage du gaz frais, à l'intérieur de la ligne 8 d'alimentation, lequel dispositif à venturi 9 est relié pneumatiquement, c'est-à-dire est mis en communication fluidique, via une ligne pneumatique 18a, avec un dispositif d'équilibrage de pression 18 comportant deux chambres internes séparées par une cloison mobile ou déformable.

Le dispositif d'équilibrage 18 est lui-même relié pneumatiquement, via une ligne d'équilibrage 18b, à ladite valve 4 de décharge.

En cas d'augmentation du débit de gaz frais dans la ligne 8, il se crée par effet venturi, une dépression au-dessus du clapet de la valve 4 de décharge pour diminuer sa résistance ; cette dépression étant relayée par le dispositif 18 d'équilibrage de pression.

La présente invention est de réduire le temps de réponse de la valve 4 à un changement de consigne de concentration du mélange grâce à un asservissement électronique ou mécanique de la pression de commande de la valve 4.

En outre, afin d'assurer un contrôle encore plus efficace de la pression dans le circuit 1 du ventilateur, on peut également incorporer audit ventilateur un dispositif de contrôle du remplissage du volume d'accumulation 12 du circuit 1.

En effet, il peut être utile, parfois, d'optimiser le débit de remplissage du réservoir 12 d'accumulation de façon à compenser juste la consommation du patient et les déperditions éventuelles du circuit, car si une fuite intervient sur le circuit, la quantité de gaz insufflée à chaque cycle respiratoire au patient est susceptible de diminuer fortement et doit donc être signalée rapidement.

Pour ce faire, on opère préférentiellement à une mesure du débit sortant du circuit par le trop plein du réservoir 12 d'accumulation, du débit entrant et sortant du réservoir d'accumulation, et/ou de la pression à l'intérieur du réservoir d'accumulation en fin -de phase expiratoire.

L'ensemble des mesures est ensuite traité par un logiciel de traitement d'informations et une information est donnée à l'utilisateur lorsque le réservoir d'accumulation n'atteint pas un seuil de remplissage minimal avant chaque début de phase inspiratoire.

De même, une information est donnée lorsque le débit à la sortie du trop plein devient trop important par rapport au débit de gaz frais réglé et peut être corrélé aux mesures de concentrations effectuées dans les gaz frais et dans les branches inspiratoires ou expiratoires ou à leurs différences, de façon à éliminer les cas où l'utilisateur règle volontairement un fort débit de gaz frais pour obtenir une rapide évolution des concentrations de son circuit.

## Revendications

1. Appareil d'anesthésie respiratoire comportant :
- circuit de gaz (1) comprenant :
(a) une branche inspiratoire (2) susceptible d'acheminer un mélange de gaz anesthésique jusqu'à des moyens de liaison (24) destinés à être reliés aux voies aériennes supérieures d'un patient (11) ;
(b) une branche expiratoire (3) susceptible d'acheminer un mélange gazeux contenant du CO₂ expiré par ledit patient (11) ;
lesdites branche inspiratoire (2) et branche expiratoire (3) formant le circuit de gaz (1) en boucle,
- au moins une ligne (8) d'alimentation en gaz frais, en communication fluide avec au moins une partie dudit circuit (1) de gaz, et destinée à alimenter ledit circuit (1) de gaz en gaz frais ;
- au moins une valve (4) de décharge à valeur de consigne de pression ajustable, agencée sur la branche inspiratoire (2) ou sur la branche expiratoire (3) ; et
- des moyens de pilotage de la valve de décharge (4) agissant sur ladite valve de décharge (4) en réponse à la détection d'une variation de débit et/ou de pression du gaz frais à l'intérieur d'au moins une partie de la ligne (8) d'alimentation en gaz frais pour ajuster la consigne de pression de ladite valve (4) de décharge.

2. Appareil selon la revendication 1, caractérisé en ce que les moyens de pilotage agissent en réponse à une variation d'au moins le débit dans au moins une partie de la ligne (8) d'alimentation.

3. Appareil selon l'une des revendications 1 ou 2, caractérisé en ce que les moyens de pilotage comprennent des moyens de mesure (7) de débit et/ou de pression agencés sur la ligne (8) d'alimentation en gaz frais et des moyens de traitement électronique (6) reliés électriquement auxdits moyens de mesure (7).

4. Appareil selon l'une des revendications 1 à 3, caractérisé en ce que les moyens de traitement (6) électronique agissent sur la valve (4) de décharge en réponse auxdits moyens de mesure (7).

5. Appareil selon l'une des revendications 1 à 4, caractérisé en ce que les moyens de traitement (6) commandent une diminution de la valeur de consigne de pression de la valve (4) de décharge en réponse à une détermination par les moyens de mesure (7), d'une augmentation de débit de gaz frais dans la ligne (8) d'alimentation.

6. Appareil selon l'une des revendications 1 à 5, caractérisé en ce que les moyens de traitement (6) agissent sur la valve (4) de décharge par l'intermédiaire d'une ligne (10) de commande de pression.

7. Appareil selon l'une des revendications 1 ou 2, caractérisé en ce que les moyens de pilotage comprennent un dispositif à venturi (9) agencé dans la ligne (8) d'alimentation en gaz frais, ledit dispositif à venturi (9) étant relié pneumatiquement (18a) à un dispositif d'équilibrage de pression (18), ledit dispositif d'équilibrage étant relié pneumatiquement (18b) à ladite valve (4) de décharge.

8. Appareil selon l'une des revendications 1,2 ou 7, caractérisé en ce que ledit dispositif d'équilibrage de pression (18) comporte au moins deux chambres internes séparées par une cloison mobile et/ou déformable.

9. Procédé de commande d'un appareil selon l'une des revendications 1 à 6, caractérisé en ce qu'on procède selon les étapes de :
(a) déterminer au moins une information de débit et/ou pression de gaz frais circulant dans la ligne (8) d'alimentation au moyen des moyens de mesure (7) de débit et/ou de pression agencés sur ladite ligne (8) ;
(b) transmettre ladite information auxdits moyens de traitement (6) électroniques et traiter ladite information ;
(c) modifier ou ajuster la valeur de pression de consigne de la valve de décharge (4).

10. Procédé selon la revendication 9, caractérisé en ce que le traitement de l'information de l'étape (b) comprend une intégration de la valeur de débit mesurée à l'étape (a) et une comparaison de cette valeur intégrée à une valeur de référence.

11. Procédé selon l'une des revendications 9 ou 10, caractérisé en ce qu'on diminue la valeur de la pression de consigne de la valve de décharge (4) lorsqu'on détermine que le débit de gaz frais mesuré augmente.
